# EUROPEAN PATENT SPECIFICATION

(11) **EP 1 742 677 B1**
(45) Date of publication and mention of the grant of the patent: **10.06.2009**
(21) Application number: 05729976.0
(22) Date of filing: 25.04.2005
(51) Int. Cl.: A61L 27/10, A61L 27/12, A61L 27/56, A61L 27/50

(54) **CERAMIC BULKING AGENT**
KERAMISCHES BULKING-MITTEL
AGENT GONFLANT CERAMIQUE

(30) Priority: 23.04.2004 ZA 200403126
(43) Date of publication of application: 17.01.2007
(73) Proprietor: Kotzé, Jacobus Adriaan Albertus, Waterkloof Ridge Extension 2 0181 Pretoria (ZA); Kotzé, Hermanus Lambertus, 3867 Mtunzini (ZA); Smyth, Stuart Kenneth Jackson, Essex Junction VT 05452 (US)
(72) Inventor: Kotzé, Jacobus Adriaan Albertus, Waterkloof Ridge Extension 2 0181 Pretoria (ZA); Kotzé, Hermanus Lambertus, 3867 Mtunzini (ZA); Smyth, Stuart Kenneth Jackson, Essex Junction VT 05452 (US)
(74) Representative: Stuttard, Garry Philip
(86) International application number: PCT/IB2005/051335
(87) International publication number: WO 2005/102405

(56) References cited:
- WO-A-01/19287
- WO-A-98/01088
- US-A- 5 336 263
- US-B1- 6 537 574
- DMOCHOWSKI R R ET AL: "Injectable agents in the treatment of stress urinary incontinence in women: Where are we now?" UROLOGY, BELLE MEAD, NJ, US, vol. 56, no. 6A Supplement, December 2000 (2000-12), pages 32-40, XP002235141 ISSN: 0090-4295

## Description

### INTRODUCTION

This invention relates to a tissue bulking agent and a device for bulking a portion of tissue *in vivo.* This invention further relates to a method and means for treating urinary incontinence related disorders. More particularly, but not exclusively, this invention relates to an injectable ceramic composite for use as a tissue bulking agent in the human body.

### BACKGROUND TO THE INVENTION

The involuntary loss of urine, known as urinary incontinence, is a significant problem affecting over 45 million men and women around the world. The failure to store urine properly is typically a result of a malfunctioning bladder or urethra. Some of the most severe cases of both male and female incontinence are those resulting from a poor or malfunctioning bladder mechanism. Incontinence may be caused by a lack of anatomic support of the urethra allowing it to move out of the abdominal cavity or by intrinsic deficiency of the urethral closure mechanism. A normal urethra will not leak at any physiologic abdominal pressure, but individuals who suffer from incontinence, may experience leakage during, for example, coughing or straining. Therefore, proper treatment of urinary incontinence is directed towards methods of achieved urethral resistance to changes in abdominal pressure.

Before 1995, when injectable collagen was introduced, there were virtually no options for the millions of women with stress incontinence other than invasive surgery. Collagen is injected into tissue, such as the urinary sphincter, to increase resistance to the flow of urine. This method suffers from some serious disadvantages. Collagen is water-soluble and is therefore absorbed by the body, so that repeated treatments are necessary. This makes the cost of collagen treatments virtually prohibitive. In addition, some patients develop allergic reactions that include rashes, swelling, erythema and anaphylactic shock. Collagen injections have also been used to augment other tissue, such as lip tissue in order to provide the lips with a filler appearance. However, this procedure has similar limitations to the collagen-treatment of incontinence, namely high cost, absorption of collagen by the body and allergic reactions to the treatment.

Another known material used to treat incontinence is carbon-coated beads. Recent double blind randomised studies comparing carbon-coated beads to collagen, however, reported only equal effectiveness or a similar poor level of performance. There was also no difference in the number of treatments between the two groups.

Within North America, materials such as collagen, carbon-coated beads and Teflon were intended to treat the millions of patients who specifically had intrinsic sphincter deficiency, where total closure of the sphincter is not achieved. Eighty two percent of the female stress incontinent patients' conditions were attributed to urethral hyper mobility, where the urethra moves or tugs on the sphincter when abdominal pressure is applied, allowing urine to leak. Until recently, bulking the sphincter has not been indicated for the treatment of hyper mobility, and leading academics report that mild to moderate stress incontinence is not entirely due to one or the other condition. It has been reported that physicians rarely see hyper mobility without intrinsic sphincter deficiency and that in fact they believe they are not separate entities, which suggests that hyper mobility is not the cause for incontinence. The outcome of their work emphasises the likelihood of achieving coaptation at the urethral outlet at rest and thus likely to improve the patient's symptoms.

Ceramic particles that have been used in the past as bulking compounds showed to be ineffective owing to the fact that the prior art particles provided insufficient binding areas where tissue could bind. A disadvantage of the known ceramic bulking compounds is therefore that the particles migrate from the area being treated.

Because of the limitations of current materials (resorption and migration), the problem of incontinence is difficult and expensive to control. There is consequently a documented need from the medical community for an injectable composite that would help control incontinence within the Pelvic Floor region/GU system.

### OBJECTS OF THE INVENTION

It is therefore an object of the present invention to provide a tissue bulking agent; a device for bulking a portion of tissue *in vivo*; and a method and means for treating incontinence with which the aforesaid disadvantages can be overcome or at least minimised. Another object of the present invention is to provide an injectable ceramic composite for use as a tissue bulking agent in the human body.

### SUMMARY OF THE INVENTION

According to a first aspect of the invention there is provided a tissue bulking agent comprising a particulate micro-porous bioinert ceramic material, having interconnected pores.

Further according to the invention the particles are in the form of spheres.

Further according to the invention the micro-pores are exposed on the surface of the spheres, hence rendering the surface of the spheres smooth on a macro-scale, but porous and uneven on a micro-scale to enhance and promote the formation of tissue in the pores and the binding of tissue to the spheres.

Yet further according to the invention, the pores on the surface of each sphere are connected to at least some of the pores inside such sphere via blow-holes and at least some of these internal pores are in turn interconnected, so that in addition to a high porosity, the spheres have a high permeability to gas and liquid.

The particles may have a porosity of between 30% and 70% per volume.

The pores of the particles may have diameters in the range of from 0.3 to 10 micrometer.

The particles may have a diameter larger than 45 micrometer, typically 110 to 600 micrometer.

The particles may be mobilised by mixing the particles with a bio-suitable carrier to render the particles injectable into the body of a patient.

Further according to the invention, the diameter size fractions of the particles do not differ more than 100 micrometer from each other in order to restrict bridging of the particles in a hypodermic needle during injection under pressure into the body of a patient, in use.

The bioinert ceramic material may be selected from the group consisting of sintered aluminium oxide (alumina); sintered zirconium oxide (zirconia); and the combination thereof.

The bioinert ceramic material may be hydroxyapatite.

The particles may be activated prior to injection by impregnation with a substance selected from the group consisting of traces of a bioactive resorbable bioceramic and serum from the blood of a patient.

The particles may be mobilised for injection by mixing the particles with a bio-suitable pharmaceutically acceptable carrier selected from the group consisting of a gel, and a viscous, lubricating liquid.

According to a second aspect of the invention there is provided a device for bulking a portion of tissue *in vivo,* comprising an effective amount of a tissue bulking agent according to a first aspect of the invention; and applying means for applying the bulking agent to the tissue.

The applying means may be in the form of a hypodermic syringe having an outlet opening of a diameter larger than 110 micrometer.

The bulking agent may be disposed inside the syringe.

According to a third aspect of the invention there is provided use of a tissue bulking agent according to the first aspect of the invention in a method for treating incontinence related disorders in a patient.

According to a fourth aspect of the invention there is provided use of a bulking agent according to the first aspect of the invention in the manufacture of a device for use in a method for treating incontinence related disorders in a patient.

According to a fifth aspect of the invention there is provided a method of treating incontinence related disorders in a patient including the step of locating an effective amount of a tissue bulking agent according to the first aspect of the invention in an affected area of the patient in need thereof.

The step of locating the bulking agent in the affected area may include the further step of injecting the bulking agent into the urinary sphincter, to increase resistance to the flow of urine.

The method may include the further step of moistening the bulking agent with blood drawn from the patient into a syringe containing the agent, such that the red blood cells accumulate on the surface of the particles and the serum is drawn into and stored inside the particles.

The method may include the further step of activating the particles prior to injection by impregnation with a substance selected from the group consisting of traces of a bioactive resorbable bioceramic and serum from the blood of a patient.

The method may include the further step of mobilising the particles for injection by mixing the particles with a bio-suitable pharmaceutically acceptable carrier selected from the group consisting of a gel, and a viscous, lubricating liquid.

According to a sixth aspect of the invention there is provided a method of bulking a portion of tissue of a human body including the step of introducing to that portion a volume of the bulking agent according to a first aspect of the invention.

The method may include the further step of injecting the bulking agent subcutaneously.

Alternatively, the method may include the further step of injecting the bulking agent into the tissue itself.

The invention will now be described further by way of a non-limiting example of a preferred embodiment of the invention.

The bulking agent according to the present invention is prepared by a method including the steps of:
- milling solid alumina (Al₂O₃) into powder (1 micrometer diameter , particles);
- adding a combustible substance known in the trade of manufacturing porous ceramic structures to the powder,
- mixing the powder with water to form a paste or slurry;
- forming solid spherical particles from the slurry by applying methods known in the art;
- sintering the spherical particles at a temperature of 1350 °C to form inert micro-porous ceramic spheres having pore sizes of between 0.3 to 10 micrometers and diameter of between 110 and 600 micrometers; and
- screening the spheres into pre-selected size ranges, with the respective diameters of spheres in a particular range not differing more than 100 micrometer from each other.

The applicants have found that a bulking agent according to the present invention meets most of the clinical requirements for the successful treatment of urinary incontinence in that the bulking agent, *inter alia:*
- is an immobile, permanent bulking agent;
- conforms to physical requirements for placement by injection ;
- is non-immunogenic, hypoallergenic and non-antigenic;
- bio-compatible, non-resorbable, with no measurable inflammatory response;
- provides satisfactory and rapid wound healing in the applied area with adequate or minimal fibrotic ingrowths;
- retains tissue bulking/enhancement and in some cases replacement over prolonged periods of time;
- when injected into the urethral sphincter of patients suffering from pressure incontinence of urine, the appropriate volume of ceramic spheres increases the intra-urethral pressure so as to render the treated patients more continent of urine;
- includes spheres having particle sizes above 100 microns and surface roughness or pores exposed on the surface that instigate adhesion to host tissue; and
- provides synthesis in functionality between a carrier and the injectable composite material.

In use, the bulking agent is disposed inside a syringe having an outlet and needle attached to the outlet with a bore having a diameter larger than 0.6 mm. The bulking agent is then activated by impregnating the spheres with traces of a bioactive resorbable bioceramic such as tricalcium phosphate. The spheres are mobilised for injection by mixing the spheres with a bio-suitable carrier gel or a viscous, lubricating carrier liquid.

Thereafter, the activated and mobilised bulking agent is injected into the tissue in the area to be treated or bulked, such as the pelvic floor or urinary sphincter.

The spheres could alternatively be activated and mobilised by the *in situ* impregnation with serum from the blood of a patient. This includes the moistening of the bulking agent with blood drawn from the patient into the syringe containing the agent, such that the red blood cells accumulate on the surface of the spheres and the serum is drawn into and stored inside the spheres. The applicants foresee that this will stimulate tissue forming on the surface even more and will reduce any imminogenic reactions to the bulking agent even further.

The applicants further foresee that the bulking agent according to the invention could be manufactured and prepared for application at a relatively low cost.

It will be appreciated that the application of the bulking agent according to the present invention is not limited to use in the treatment of incontinence related disorders, but could be applied anywhere in the human body where a bulking agent is required, such as in reconstructive surgery following damage to tissue; and in cosmetic surgery where the volume of certain body parts such as lips are to be increased.

The characteristics of the bulking agent of the present invention, such as, *inter alia,* the low cost of manufacture; the safety; stability; immobility after application; and the ease of application thereof, not only overcomes the disadvantages of the prior art, but also renders the bulking agent and the method of the invention suitable and available to a wide range of patients including those suffering from incontinence related disorders.

The description of the embodiments of the present invention is given above for the understanding of the present invention. It will be appreciated further that the invention is not limited to the particular embodiments described herein, but is capable of various modifications and rearrangements as will be apparent to those skilled in the art without departing from the scope of the appended claims.

## Claims

1. A tissue bulking agent comprising a particulate micro-porous bioinert ceramic material, having interconnected pores.

2. A tissue bulking agent according to claim 1 wherein the particles are in the form of spheres.

3. A tissue bulking agent according to claim 2 wherein the micro-pores are exposed on the surface of the spheres, hence rendering the surface of the spheres smooth on a macro-scale, but porous and uneven on a micro-scale to enhance and promote the formation of tissue in the pores and the binding of tissue to the spheres.

4. A tissue bulking agent according to claim 3 wherein the pores on the surface of each sphere are connected to at least some of the pores inside such sphere via blow-holes and at least some of these internal pores are in turn interconnected, so that in addition to a high porosity, the spheres have a high permeability to gas and liquid.

5. A tissue bulking agent according to any one of claims 1 to 4 wherein the particles have a porosity of between 30% and 70% per volume.

6. A tissue bulking agent according to any one of claims 1 to 5 wherein the pores of the particles have diameters in the range of from 0.3 to 10 micrometer.

7. A tissue bulking agent according to any one of claims 1 to 6 wherein the particles have a diameter larger than 45 micrometer.

8. A tissue bulking agent according to claim 7 wherein the particles have a diameter of 110 to 600 micrometer.

9. A tissue bulking agent according to any one of the preceding claims wherein the particles are mobilised by mixing the particles with a bio-suitable carrier to render the particles injectable into the body of a patient.

10. A tissue bulking agent according to claim 9 wherein the particles are mobilised for injection by mixing the particles with a bio-suitable pharmaceutically acceptable carrier selected from the group consisting of a gel, and a viscous, lubricating liquid.

11. A tissue bulking agent according to claim 9 or 10 wherein the diameter size fractions of the particles do not differ more than 100 micrometer from each other in order to restrict bridging of the particles in a in a hypodermic needle during injection under pressure into the body of a patient, in use.

12. A tissue bulking agent according to any one of claims 9 to 11 wherein the particles are activated prior to injection by impregnation with a substance selected from the group consisting of traces of a bioactive resorbable bioceramic and serum from the blood of a patient.

13. A tissue bulking agent according to any one of the preceding claims wherein the bioinert ceramic material is selected from the group consisting of sintered aluminium oxide (alumina); sintered zirconium oxide (zirconia); and the combination thereof.

14. A tissue bulking agent according to any one of the preceding claims wherein the bioinert ceramic material is hydroxyapatite.

15. A device for bulking a portion of tissue *in vivo,* comprising an effective amount of a tissue bulking agent according to claims 1 to 14; and applying means for applying the bulking agent to the tissue.

16. A device according to claim 15 wherein the applying means is in the form of a hypodermic syringe having an outlet opening of a diameter larger than 110 micrometer.

17. A device according to claim 16 wherein the bulking agent is disposed inside the syringe.

18. A tissue bulking agent according to claims 1 to 14 for use in the treatment of incontinence related disorders in a patient.

19. A tissue bulking agent for use in the treatment of incontinence related disorders in a patient as claimed in claim 18 wherein an effective amount of the tissue bulking agent is located in an affected area of the patient.

20. A tissue bulking agent for use in the treatment of incontinence related disorders in a patient as claimed in claim 19 wherein the step of locating the bulking agent in the affected area includes the further step of injecting the bulking agent into the urinary sphincter, to increase resistance to the flow of urine.

21. A tissue bulking agent for use in the treatment of incontinence related disorders in a patient as claimed in claim 19 or claim 20 which includes the further step of moistening the bulking agent with blood drawn from the patient into a syringe containing the agent, such that the red blood cells accumulate on the surface of the particles and the serum is drawn into and stored inside the particles.

22. A tissue bulking agent for use in the treatment of incontinence related disorders in a patient as claimed in claim 21 which includes the further step of activating the particles prior to injection by impregnation with a substance selected from the group consisting of traces of a bioactive resorbable bioceramic and serum from the blood of a patient.

23. A tissue bulking agent for use in the treatment of incontinence related disorders in a patient as claimed in claim 22 which includes the further step of mobilising the particles for injection by mixing the particles with a bio-suitable pharmaceutically acceptable carrier selected from the group consisting of a gel, and a viscous, lubricating liquid.

24. Use of a tissue bulking agent according to claims 1 to 14 in the manufacture of a device for use in a method for treating incontinence related disorders in a patient.

25. Use according to claim 24 wherein an effective amount of the tissue bulking agent is located in an affected area of the patient.

26. Use according to claim 25 wherein the step of locating the bulking agent in the affected area includes the further step of injecting the bulking agent into the urinary sphincter, to increase resistance to the flow of urine.

27. Use according to claim 25 or claim 26 which includes the further step of moistening the bulking agent with blood drawn from the patient into a syringe containing the agent, such that the red blood cells accumulate on the surface of the particles and the serum is drawn into and stored inside the particles.

28. Use according to claim 27 which includes the further step of activating the particles prior to injection by impregnation with a substance selected from the group consisting of traces of a bioactive resorbable bioceramic and serum from the blood of a patient.

29. Use according to claim 28 which includes the further step of mobilising the particles for injection by mixing the particles with a bio-suitable pharmaceutically acceptable carrier selected from the group consisting of a gel, and a viscous, lubricating liquid.

30. A tissue bulking agent according to claims 1 to 14 for use in bulking a portion of tissue of a human body, by being introduced into the tissue of the human body.

31. A tissue bulking agent for use in bulking a portion of tissue of a human body according to claim 30 which is injected subcutaneously.

32. A tissue bulking agent for use in bulking a portion of tissue of a human body according to claim 30 which is injected into the tissue itself.

33. Use of a tissue bulking agent according to claims 1 to 14 in the preparation of a medicament for use in the treatment of incontinence related disorders in a patient.

34. Use of the tissue bulking agent in the preparation of a medicament for use in the treatment of incontinence related disorders in a patient as claimed in claim 33 wherein an effective amount of the tissue bulking agent is located in an affected area of the patient.

35. Use of the tissue bulking agent in the preparation of a medicament for use in the treatment of incontinence related disorders in a patient as claimed in claim 34 wherein the step of locating the bulking agent in the affected area includes the further step of injecting the bulking agent into the urinary sphincter, to increase resistance to the flow of urine.

36. Use of the tissue bulking agent in the preparation of a medicament for use in the treatment of incontinence related disorders in a patient as claimed in claim claim 34 or claim 35 which includes the further step of moistening the bulking agent with blood drawn from the patient into a syringe containing the agent, such that the red blood cells accumulate on the surface of the particles and the serum is drawn into and stored inside the particles.

37. Use of the tissue bulking agent in the preparation of a medicament for use in the treatment of incontinence related disorders in a patient as claimed in claim 36 which includes the further step of activating the particles prior to injection by impregnation with a substance selected from the group consisting of traces of a bioactive resorbable bioceramic and serum from the blood of a patient.

38. Use of the tissue bulking agent in the preparation of a medicament for use in the treatment of incontinence related disorders in a patient as claimed in claim 37 which includes the further step of mobilising the particles for injection by mixing the particles with a bio-suitable pharmaceutically acceptable carrier selected from the group consisting of a gel, and a viscous, lubricating liquid.

39. Use of a tissue bulking agent according to claims 1 to 14 in the preparation of a medicament for use in bulking a portion of tissue of a human body by being introduced into the tissue of the human body.

40. Use of the tissue bulking agent in the preparation of a medicament for use in bulking a portion of tissue of a human body as claimed in claim 39 by being injected subcutaneously.

41. Use of the tissue bulking agent in the preparation of a medicament for use in bulking a portion of tissue of a human body as claimed in claim 39 by being injected into the tissue itself.

## Patentansprüche

1. Gewebe-Bulking Agent, das ein partikuläres mikroporöses bioinertes Keramikmaterial, das miteinander verbundene Poren hat, umfasst.

2. Gewebe-Bulking Agent gemäß Anspruch 1, in dem die Partikel in der Form von Sphären sind.

3. Gewebe-Bulking Agent gemäß Anspruch 2, wobei die Mikroporen an der Oberfläche der Sphären exponiert sind, wodurch die Oberfläche der Sphären im Makromaßstab glatt gemacht ist, allerdings porös und uneben im Mikromaßstab gemacht ist, um die Bildung von Gewebe in den Poren und die Bindung von Gewebe an die Sphären zu verstärken und zu begünstigen.

4. Gewebe-Bulking Agent gemäß Anspruch 3, wobei Poren an der Oberfläche jeder Sphäre mit wenigstens einigen der Poren im Inneren einer solchen Sphäre via Lunker (blow-holes) verbunden sind und wenigstens einige dieser internen Poren wiederum miteinander verbunden sind, sodass die Sphären zusätzlich zu einer hohen Porosität eine hohe Permeabilität für Gas und Flüssigkeit haben.

5. Gewebe-Bulking Agent gemäß einem der Ansprüche 1 bis 4, wobei die Partikel eine Porosität von zwischen 30% und 70% pro Volumen haben.

6. Gewebe-Bulking Agent gemäß einem der Ansprüche 1 bis 5, wobei die Poren der Partikel Durchmesser im Bereich von 0.3 bis 10 Mikrometer haben.

7. Gewebe-Bulking Agent gemäß einem der Ansprüche 1 bis 6, wobei die Partikel einen Durchmesser von größer als 45 Mikrometer haben.

8. Gewebe-Bulking Agent gemäß Anspruch 7, wobei die Partikel einen Durchmesser von 110 bis 600 Mikrometer haben.

9. Gewebe-Bulking Agent gemäß einem der vorangehenden Ansprüche, wobei die Partikel mobilisiert werden, indem die Partikel mit einem biologisch geeigneten Träger vermischt werden, um die Partikel in den Körper eines Patienten injizierbar zu machen.

10. Gewebe-Bulking Agent gemäß Anspruch 9, wobei die Partikel zur Injektion mobilisiert werden, indem die Partikel mit einem biologisch geeigneten pharmazeutisch verträglichen Träger, ausgewählt aus der Gruppe, bestehend aus einem Gel und einer viskosen gleitend machenden Flüssigkeit, vermischt werden.

11. Gewebe-Bulking Agent gemäß Anspruch 9 oder 10, wobei die Durchmessergrößenfraktionen der Partikel um nicht mehr als 100 Mikrometer voneinander differieren, um eine Brückenbildung der Partikel in einer hypodermen Nadel während Injektion unter Druck in den Körper eines Patienten zu beschränken.

12. Gewebe-Bulking Agent gemäß einem der Ansprüche 9 bis 11, wobei die Partikel vor Injektion durch Imprägnierung mit einer Substanz, ausgewählt aus der Gruppe, bestehend aus Spuren einer bioaktiven resorbierbaren Biokeramik und Serum aus dem Blut eines Patienten, aktiviert werden.

13. Gewebe-Bulking Agent gemäß einem der vorangehenden Ansprüche, wobei das bioinerte keramische Material ausgewählt ist aus der Gruppe, bestehend aus gesintertem Aluminiumoxid (Alumina); gesintertem Zirkoniumoxid (Zirkonia) und der Kombination davon.

14. Gewebe-Bulking Agent gemäß einem der vorangehenden Ansprüche, wobei das bioinerte keramische Material Hydroxyapatit ist.

15. Vorrichtung zur Volumenvergrößerung/zum Auffüllen eines Gewebeteils *in vivo,* umfassend eine wirksame Menge eines Gewebe-Bulking Agent gemäß den Ansprüchen 1 bis 14 und ein Anwendungsmittel zum Anwenden/Applizieren des Bulking Agent auf das Gewebe.

16. Vorrichtung gemäß Anspruch 15, wobei das Anwendungsmittel in der Form einer hypodermen Spritze ist, die eine Auslassöffnung mit einem Durchmesser von größer als 110 Mikrometer hat.

17. Vorrichtung gemäß Anspruch 16, wobei das Bulking Agent im Inneren der Spritze angeordnet ist.

18. Gewebe-Bulking Agent gemäß den Ansprüchen 1 bis 14 zur Verwendung in der Behandlung von mit Inkontinenz in Beziehung stehenden Störungen bei einem Patienten.

19. Gewebe-Bulking Agent zur Verwendung in der Behandlung von mit Inkontinenz in Beziehung stehenden Störungen bei einem Patienten, wie in Anspruch 18 beansprucht, wobei eine wirksame Menge des Gewebe-Bulking Agent in einem betroffenen Bereich des Patienten lokalisiert wird.

20. Gewebe-Bulking Agent zur Verwendung in der Behandlung von mit Inkontinenz in Beziehung stehenden Störungen bei einem Patienten, wie in Anspruch 19 beansprucht, wobei der Schritt des Lokalisierens des Bulking Agent in dem betroffenen Bereich den weiteren Schritt des Injizierens des Bulking Agent in den Blasenschließmuskel umfasst, um den Widerstand gegen den Urinstrom zu erhöhen.

21. Gewebe-Bulking Agent zur Verwendung in der Behandlung von mit Inkontinenz in Beziehung stehenden Störungen bei einem Patienten, wie in Anspruch 19 oder 20 beansprucht, umfassend den weiteren Schritt des Befeuchtens des Bulking Agent mit Blut, das aus dem Patienten in eine Spritze, die das Agent enthält, gezogen wird, sodass die roten Blutzellen an der Oberfläche der Partikel akkumulieren und das Serum in das Innere der Partikel gezogen und dort gespeichert wird.

22. Gewebe-Bulking Agent zur Verwendung in der Behandlung von mit Inkontinenz in Beziehung stehenden Störungen bei einem Patienten, wie in Anspruch 21 beansprucht, umfassend den weiteren Schritt der Aktivierung der Partikel vor Injektion durch Imprägnierung mit einer Substanz, ausgewählt aus der Gruppe, bestehend aus Spuren einer bioaktiven resorbierbaren Biokeramik und Serum aus dem Blut eines Patienten.

23. Gewebe-Bulking Agent zur Verwendung in der Behandlung von mit Inkontinenz in Beziehung stehenden Störungen bei einem Patienten, wie in Anspruch 22 beansprucht, umfassend den weiteren Schritt der Mobilisierung der Partikel zur Injektion durch Mischen der Partikel mit einem biologisch geeigneten pharmazeutisch verträglichen Träger, ausgewählt aus der Gruppe, bestehend aus einem Gel und einer viskosen gleitend machenden Flüssigkeit.

24. Verwendung eines Gewebe-Bulking Agent gemäß den Ansprüche 1 bis 14 bei der Herstellung einer Vorrichtung zur Verwendung in einem Verfahren zur Behandlung von mit Inkontinenz in Beziehung stehenden Störungen bei einem Patienten.

25. Verwendung gemäß Anspruch 24, wobei eine wirksame Menge des Gewebe-Bulking Agent in einem betroffenen Bereich des Patienten lokalisiert wird.

26. Verwendung gemäß Anspruch 25, wobei der Schritt des Lokalisierens des Bulking Agent in dem betroffenen Bereich den weiteren Schritt des Injizierens des Bulking Agent in den Blasenschließmuskel umfasst, um den Widerstand gegenüber dem Urinstrom zu erhöhen.

27. Verwendung gemäß Anspruch 25 oder 26, umfassend den weiteren Schritt des Befeuchtens des Bulking Agent mit Blut, das aus dem Patienten in eine Spritze, die das Agent enthält, aufgezogen wird, sodass die roten Blutzellen an der Oberfläche der Partikel akkumulieren und das Serum in das Innere der Partikel gezogen und dort gespeichert wird.

28. Verwendung gemäß Anspruch 27, welche den weiteren Schritt der Aktivierung der Partikel vor Injektion durch Imprägnierung mit einer Substanz, ausgewählt aus der Gruppe, bestehend aus Spuren einer bioaktiven resorbierbaren Biokeramik und Serum aus dem Blut eines Patienten, umfasst.

29. Verwendung gemäß Anspruch 28, die den weiteren Schritt der Mobilisierung der Partikel zur Injektion durch Vermischen der Partikel mit einem biologisch geeigneten, pharmazeutisch verträglichen Träger, ausgewählt aus der Gruppe, bestehend aus einem Gel und einer viskosen gleitend machenden Flüssigkeit, umfasst.

30. Gewebe-Bulking Agent gemäß den Ansprüchen 1 bis 14 zur Verwendung bei der Volumenvergrößerung/Auffüllung eines Gewebeteils eines menschlichen Körpers, indem es in das Gewebe des menschlichen Körpers eingeführt wird.

31. Gewebe-Bulking Agent zur Verwendung bei der Volumenvergrößerung/Auffüllung eines Gewebeteils eines menschlichen Körpers gemäß Anspruch 30, das subkutan injiziert wird.

32. Gewebe-Bulking Agent zur Verwendung bei der Volumenvergrößerung/Auffüllung eines Gewebeteils eines menschlichen Körpers gemäß Anspruch 30, das in das Gewebe selbst injiziert wird.

33. Verwendung eines Gewebe-Bulking Agent gemäß den Ansprüchen 1 bis 14 in der Herstellung eines Medikaments zur Verwendung in der Behandlung von mit Inkontinenz in Beziehung stehenden Störungen bei einem Patienten.

34. Verwendung des Gewebe-Bulking Agent in der Herstellung eines Medikaments zur Verwendung in der Behandlung von mit Inkontinenz in Beziehung stehenden Störungen bei einem Patienten, wie in Anspruch 33 beansprucht, wobei eine wirksame Menge des Gewebe-Bulking Agent in einem betroffenen Bereich des Patienten lokalisiert wird.

35. Verwendung des Gewebe-Bulking Agent in der Herstellung eines Medikaments zur Verwendung in der Behandlung von mit Inkontinenz in Beziehung stehenden Störungen bei einem Patienten, wie in Anspruch 34 beansprucht, wobei der Schritt des Lokalisierens des Bulking Agent in dem betroffenen Bereich den weiteren Schritt des Injizieren des Bulking Agent in den Blasenschließmuskel umfasst, um den Widerstand gegen den Urinstrom zu erhöhen.

36. Verwendung des Gewebe-Bulking Agent in der Herstellung eines Medikaments zur Verwendung in der Behandlung von mit Inkontinenz in Beziehung stehenden Störungen bei einem Patienten, wie in Anspruch 34 oder Anspruch 35 beansprucht, welche außerdem den Schritt des Befeuchtens des Bulking Agent mit Blut, das aus dem Patienten in eine Spritze, die das Agent enthält, aufgezogen wird, umfasst, sodass die roten Blutzellen an der Oberfläche der Partikel akkumulieren und das Serum in das Innere der Partikel gezogen und dort gespeichert wird.

37. Verwendung des Gewebe-Bulking Agent in der Herstellung eines Medikaments zur Verwendung in der Behandlung von mit Inkontinenz in Beziehung stehenden Störungen bei einem Patienten, wie in Anspruch 36 beansprucht, welche den weiteren Schritt der Aktivierung der Partikel vor Injektion durch Imprägnierung mit einer Substanz, ausgewählt aus der Gruppe von Spuren einer bioaktiven resorbierbaren Biokeramik und Serum aus dem Blut eines Patienten, umfasst.

38. Verwendung des Gewebe-Bulking Agent in der Herstellung eines Medikaments zur Verwendung in der Behandlung von mit Inkontinenz in Beziehung stehenden Störungen bei einem Patienten, wie in Anspruch 37 beansprucht, welche außerdem den Schritt der Mobilisierung der Partikel zur Injektion durch Vermischen der Partikel mit einem biologisch geeigneten pharmazeutisch verträglichen Träger, ausgewählt aus der Gruppe, bestehend aus einem Gel und einer viskosen gleitend machenden Flüssigkeit, umfasst.

39. Verwendung eines Gewebe-Bulking Agent gemäß den Ansprüchen 1 bis 14 in der Herstellung eines Medikaments zur Verwendung bei der Volumenvergrößerung/Auffüllung eines Gewebeteils eines menschlichen Körpers, das in das Gewebe des menschlichen Körpers eingeführt wird.

40. Verwendung des Gewebe-Bulking Agent in der Herstellung eines Medikaments zur Verwendung bei der Volumenvergrößerung eines Gewebeteils eines menschlichen Körpers, wie in Anspruch 39 beansprucht, indem es subkutan injiziert wird.

41. Verwendung des Gewebe-Bulking Agent in der Herstellung eines Medikaments zur Verwendung bei der Volumenvergrößerung eines Gewebeteils eines menschlichen Körpers, wie in Anspruch 39 beansprucht, indem es in das Gewebe selbst injiziert wird.

## Revendications

1. Agent gonflant tissulaire comprenant un matériau céramique biologiquement inerte, microporeux particulaire ayant des pores interconnectés.

2. Agent gonflant tissulaire selon la revendication 1, dans lequel les particules se présentent sous forme de sphères.

3. Agent gonflant tissulaire selon la revendication 2, dans lequel les micropores sont exposés sur la surface des sphères, rendant ainsi la surface des sphères lisse sur une macro-échelle, mais poreuse et non uniforme sur une micro-échelle pour renforcer et favoriser la formation de tissu dans les pores et la liaison de tissu aux sphères.

4. Agent gonflant tissulaire selon la revendication 3, dans lequel les pores sur la surface de chaque sphère sont reliés à au moins une partie des pores à l'intérieur de cette sphère par des trous soufflés et au moins une partie de ces pores internes sont elles-mêmes interconnectées de sorte qu'en plus d'une porosité élevée, les sphères ont une perméabilité élevée aux gaz et aux liquides.

5. Agent gonflant tissulaire selon l'une quelconque des revendications 1 à 4, dans lequel les particules ont une porosité entre 30 % et 70 % par volume.

6. Agent gonflant tissulaire selon l'une quelconque des revendications 1 à 5, dans lequel les pores des particules ont des diamètres de l'ordre de 0,3 à 10 microns.

7. Agent gonflant tissulaire selon l'une quelconque des revendications 1 à 6, dans lequel les particules ont un diamètre supérieur à 45 microns.

8. Agent gonflant tissulaire selon la revendication 7, dans lequel les particules ont un diamètre de 110 à 600 microns.

9. Agent gonflant tissulaire selon l'une quelconque des revendications précédentes, dans lequel les particules sont mobilisées en mélangeant les particules avec un véhicule approprié biologiquement pour rendre les particules injectables dans le corps d'un patient.

10. Agent gonflant tissulaire selon la revendication 9, dans lequel les particules sont mobilisées pour injection en mélangeant les particules à un véhicule pharmaceutiquement acceptable approprié biologiquement, choisi dans le groupe comprenant un gel et un liquide lubrifiant visqueux.

11. Agent gonflant tissulaire selon la revendication 9 ou 10, dans lequel les fractions des diamètres des particules ne diffèrent pas de plus de 100 microns les unes des autres afin de limiter le pontage des particules dans une aiguille hypodermique pendant l'injection sous pression dans le corps d'un patient, au cours de l'utilisation.

12. Agent gonflant tissulaire selon l'une quelconque des revendications 9 à 11, dans lequel les particules sont activées avant injection par imprégnation avec une substance choisie dans le groupe comprenant des traces d'une biocéramique résorbable bioactive et du sérum du sang d'un patient.

13. Agent gonflant tissulaire selon l'une quelconque des revendications précédentes, dans lequel le matériau céramique biologiquement inerte est choisi dans le groupe comprenant l'oxyde d'aluminium fritté (alumine) ; l'oxyde de zirconium fritté (zircone) ; et leur combinaison.

14. Agent gonflant tissulaire selon l'une quelconque des revendications précédentes, dans lequel le matériau céramique biologiquement inerte est l'hydroxyapatite.

15. Dispositif pour faire gonfler une partie d'un tissu *in vivo,* comprenant une quantité efficace d'un agent gonflant tissulaire selon les revendications 1 à 14 ; et des moyens d'application pour appliquer l'agent gonflant au tissu.

16. Dispositif selon la revendication 15, dans lequel le moyen d'application se présente sous la forme d'une seringue hypodermique ayant une ouverture de sortie présentant un diamètre supérieur à 110 microns.

17. Dispositif selon la revendication 16, dans lequel l'agent gonflant est disposé à l'intérieur de la seringue.

18. Agent gonflant tissulaire selon les revendications 1 à 14, à utiliser dans le traitement des troubles liés à l'incontinence chez un patient.

19. Agent gonflant tissulaire à utiliser dans le traitement des troubles liés à l'incontinence chez un patient selon la revendication 18, dans lequel une quantité efficace d'agent gonflant tissulaire est située dans une zone affectée du patient.

20. Agent gonflant tissulaire à utiliser dans le traitement des troubles liés à l'incontinence chez un patient selon la revendication 19, dans lequel l'étape de placement de l'agent gonflant dans la zone affectée comprend l'étape suivante d'injection de l'agent gonflant dans le sphincter urinaire, pour augmenter la résistance à l'écoulement de l'urine.

21. Agent gonflant tissulaire à utiliser dans le traitement des troubles liés à l'incontinence chez un patient selon la revendication 19 ou la revendication 20, qui comprend l'étape suivante d'humidification de l'agent moussant avec du sang prélevé du patient dans une seringue contenant l'agent de sorte que les globules rouges s'accumulent à la surface des particules et du sérum est prélevé dans les particules et stocké à l'intérieur de celles-ci.

22. Agent gonflant tissulaire à utiliser dans le traitement des troubles associés à l'incontinence chez un patient selon la revendication 21, qui comprend l'étape suivante d'activation des particules avant injection par imprégnation avec une substance choisie dans le groupe comprenant des traces de biocéramique résorbable biologiquement active et de sérum du sang d'un patient.

23. Agent gonflant tissulaire à utiliser dans le traitement des troubles liés à l'incontinence chez un patient selon la revendication 22, qui comprend l'étape suivante de mobilisation des particules pour l'injection par mélange des particules avec un véhicule pharmaceutiquement acceptable approprié biologiquement, choisi dans le groupe comprenant un gel et un liquide lubrifiant visqueux.

24. Utilisation d'un agent gonflant tissulaire selon les revendications 1 à 14, dans la production d'un dispositif à utiliser dans un procédé de traitement des troubles liés à l'incontinence chez un patient.

25. Utilisation selon la revendication 24, dans laquelle une quantité efficace d'agent gonflant tissulaire est située dans une zone affectée du patient.

26. Utilisation selon la revendication 25, dans laquelle l'étape de placement de l'agent gonflant dans la zone affectée comprend l'étape suivante d'injection de l'agent gonflant dans le sphincter urinaire, pour augmenter la résistance à l'écoulement de l'urine.

27. Utilisation selon la revendication 25 ou la revendication 26 qui comprend l'étape suivante d'humidification de l'agent moussant avec du sang prélevé du patient dans une seringue contenant l'agent de sorte que les globules rouges s'accumulent à la surface des particules et du sérum est prélevé dans les particules et stocké à l'intérieur de celles-ci.

28. Utilisation selon la revendication 27, qui comprend l'étape suivant d'activation des particules avant injection par imprégnation avec une substance choisie dans le groupe comprenant des traces d'une biocéramique résorbable bioactive et du sérum du sang d'un patient.

29. Utilisation selon la revendication 28, qui comprend l'étape suivante de mobilisation des particules pour injection en mélangeant les particules à un véhicule pharmaceutiquement acceptable approprié biologiquement, choisi dans le groupe comprenant un gel et un liquide lubrifiant visqueux.

30. Agent gonflant tissulaire selon les revendications 1 à 14, à utiliser pour faire gonfler une partie des tissus d'un corps humain en étant introduit dans le tissu du corps humain.

31. Agent gonflant tissulaire à utiliser pour faire gonfler une partie d'un tissu d'un corps humain selon la revendication 30, qui est injecté par voie sous-cutanée.

32. Agent gonflant tissulaire à utiliser pour faire gonfler une partie d'un tissu d'un corps humain selon la revendication 30, qui est injecté dans le tissu lui-même.

33. Utilisation d'un agent gonflant tissulaire selon les revendications 1 à 14, dans la préparation d'un médicament à utiliser dans le traitement des troubles liés à l'incontinence chez un patient.

34. Utilisation de l'agent gonflant tissulaire dans la préparation d'un médicament à utiliser dans le traitement des troubles liés à l'incontinence chez un patient selon la revendication 33, dans laquelle une quantité efficace d'agent gonflant tissulaire est située dans une zone affectée du patient.

35. Utilisation de l'agent gonflant tissulaire dans la préparation d'un médicament à utiliser dans le traitement des troubles liés à l'incontinence chez un patient selon la revendication 34, dans laquelle l'étape de placement de l'agent gonflant dans la zone affectée comprend l'étape suivante d'injection de l'agent gonflant dans le sphincter urinaire, pour augmenter la résistance à l'écoulement d'urine.

36. Utilisation de l'agent gonflant tissulaire dans la préparation d'un médicament à utiliser dans le traitement des troubles liés à l'incontinence chez un patient selon la revendication 34 ou la revendication 35, qui comprend l'étape suivante d'humidification de l'agent moussant avec du sang prélevé du patient dans une seringue contenant l'agent de sorte que les globules rouges s'accumulent à la surface des particules et du sérum est prélevé dans les particules et stocké à l'intérieur de celles-ci.

37. Utilisation de l'agent gonflant tissulaire dans la préparation d'un médicament à utiliser dans le traitement des troubles liés à l'incontinence chez un patient selon la revendication 36, qui comprend l'étape suivante d'activation des particules avant injection par imprégnation avec une substance choisie dans le groupe comprenant des traces de biocéramique résorbable biologiquement active et de sérum du sang d'un patient.

38. Utilisation de l'agent gonflant tissulaire dans la préparation d'un médicament à utiliser dans le traitement des troubles liés à l'incontinence chez un patient selon la revendication 37, qui comprend l'étape suivante de mobilisation des particules pour l'injection par mélange des particules avec un véhicule pharmaceutiquement acceptable approprié biologiquement, choisi dans le groupe comprenant un gel et un liquide lubrifiant visqueux.

39. Utilisation de l'agent gonflant tissulaire selon les revendications 1 à 14, dans la préparation d'un médicament à utiliser pour faire gonfler une partie du tissu d'un corps humain en étant introduit dans le tissu du corps humain.

40. Utilisation de l'agent gonflant tissulaire dans la préparation d'un médicament à utiliser pour faire gonfler une partie du tissu d'un corps humain selon la revendication 39 en étant injecté par voie sous-cutanée.

41. Utilisation de l'agent gonflant tissulaire dans la préparation d'un médicament à utiliser pour faire gonfler une partie du tissu d'un corps humain selon la revendication 39 en étant injecté dans le tissu lui-même.
